# EUROPÄISCHE PATENTSCHRIFT

(11) **EP 0 567 673 B1**
(45) Veröffentlichungstag und Bekanntmachung des Hinweises auf die Patenterteilung: **05.07.1995**
(21) Anmeldenummer: 92107261.7
(22) Anmeldetag: 29.04.1992
(51) Int. Cl.: A61F 5/01

(54) **Kniegelenkorthese**
Knee joint orthesis
Orthèse de l'articulation du genou

(43) Veröffentlichungstag der Anmeldung: 03.11.1993
(73) Patentinhaber: Otto Bock Orthopädische Industrie Besitz- und Verwaltungs-Kommanditgesellschaft, 37115 Duderstadt (DE)
(72) Erfinder: Grifka, I., Dr. med., W-4630 Bochum (DE)
(74) Vertreter: Gramm, Werner, Prof., Dipl.-Ing.

(56) Entgegenhaltungen:
- EP-A- 0 356 623
- DE-A- 4 013 693
- DE-U- 8 517 061
- FR-A- 2 441 382
- US-A- 2 460 895
- US-A- 4 632 097

## Beschreibung

Die Erfindung betrifft eine Kniegelenkorthese mit einem über Bandagen oder dergleichen am Oberschenkel festzulegenden Oberschenkelteil, einem ebenfalls über Bandagen oder dergleichen am Unterschenkel festzulegenden Unterschenkelteil, zwei sich seitlich gegenüberliegenden, die beiden Schenkelteile gelenkig miteinander verbindenden Gelenkschienen, die teleskopförmig längenveränderlich ausgebildet und mit ihrem oberen Ende am Oberschenkelteil angelenkt sind und mit einer eine Dorsalierung des Unterschenkels bewirkenden Einrichtung.

Eine derartige Ausführungsform läßt sich der EP 0 356 623 A1 entnehmen. Dabei ist jede Gelenkschiene mit ihrem oberen Ende am unteren Ende einer Stange angelenkt, die ihrerseits mit ihrem oberen Ende am Oberschenkelteil angelenkt ist und um diesen Gelenkpunkt so nach vorn bzw. hinten verstellbar ist, daß das durch den oberen Anlenkpunkt der Gelenkschienen definierte Gelenk vor (anterior) oder hinter (posterior) der Kniegelenkachse liegt. Je nach Einstellung dieser Stange entsteht somit eine nach vorne oder eine nach hinten (Dorsalierung) wirkende Kraft auf den Unterschenkel.

Bei dieser vorbekannten Ausführungsform ist jede Gelenkschiene am Unterschenkelteil in einer hier starr festgelegten Teleskopführung axial verschiebbar.

Eine Kniegelenkorthese mit einem über Bandagen am Oberschenkel festzulegenden Oberschenkelteil, einem in gleicher Weise am Unterschenkelteil festzulegenden Unterschenkelteil und zwei sich seitlich gegenüberliegenden, die beiden Schenkelteile gelenkig miteinander verbindenden Gelenkschienen läßt sich auch der DE 40 13 693 C2 entnehmen. Diese vorbekannte Kniegelenkorthese besteht aus zwei seitlich gegenüberliegend am Ober- und Unterschenkel mittels Bandagen befestigbaren Gelenkschienen, die von je einer Oberschenkel- und Unterschenkelschiene gebildet sind, die über ein Gelenk miteinander verbunden sind. An den freien Enden der Gelenkschienen sind je zwei auf der Vorderseite des Beines parallel und auf der Rückseite des Beines sich kreuzend angeordnete Seilzüge längenverstellbar gehalten. Dabei sind an den Oberschenkelschienen sowie den Unterschenkelschienen zwei bandförmige Querverbinder gehalten, die Führungsschlitze aufweisen, durch die sich die parallelen Seilzüge erstrecken.

Bei derartigen sogenannten funktionellen Orthesen wird versucht, die innen- und außenseitig am Kniegelenk differente und individuell unterschiedliche Gelenkmechanik auf eine biomechanische Vereinfachung zu reduzieren, die sich auf den Verlauf des vorderen und hinteren Kreuzbandes stützt und ihren Ausfluß in speziellen Gelenken findet. Hierbei wird nicht nur der Einfluß der übrigen Bandstrukturen vernachlässigt. Es bleiben auch die innen- und außenseitig am Kniegelenk unterschiedlichen Bewegungsabläufe unberücksichtigt; das Kniegelenk wird in eine Zwangsführung gebracht. Der niemals aufeinander abzustimmende Bewegungsablauf von gelenkgeführter Orthese und Kniegelenk zeigt sich in Scherbewegungen der Orthesenteile, wobei die Hebelkräfte der Schienenarme zum einen auf die Kniegelenkbewegung Einfluß nehmen, zum anderen aber Scheuerbereiche an der Haut provozieren. Die üblicherweise verwendeten Gurtungen zur Adaption der Schienenteile am Ober- und Unterschenkel führen zu zusätzlichen Einschnürungen und insbesondere im Bereich der Kniekehle zur Bewegungseinschränkung.

Nachteilig bei den vorbekannten Kniegelenkorthesen ist das sehr umständliche Anlegen der Orthese und ihr zum Teil hohes Gewicht.

Der Erfindung liegt die Aufgabe zugrunde, die eingangs beschriebene Kniegelenkorthese in ihrer Funktion sowie in konstruktiver Hinsicht zu verbessern.

Diese Aufgabe wird erfindungsgemäß dadurch gelöst, daß jede der beiden Gelenkschienen mit ihrem unteren Ende am Unterschenkelteil angelenkt ist und daß die genannte Einrichtung derart gestaltet ist, daß die Dorsalierung über eine Rückverlagerung der Gelenkschienen bewirkt wird.

Ein wesentliches Merkmal der erfindungsgemäßen Orthese ist somit darin zu sehen, daß kein mechanisches, die individuelle Kniegelenkmechanik nachahmendes Gelenk mehr vorgesehen ist.

Vielmehr wird der Kniegelenkmechanik freier Raum gelassen, um die physiologische Bewegung ungehindert durchführen zukönnen. Der jeweilige Bewegungsmittelpunkt, mit dem auch seitlich unterschiedlichen Verlauf der Gelenkachse, kann sich entsprechend den individuellen anatomischen Verhältnissen zwischen den Anlenkpunkten der Gelenkschienen einstellen. Durch die Verbindung von Oberschenkel- und Unterschenkelteil der Orthese wird ein Abhebeln oder Verrutschen und Scheuern aufgrund einer eigenständigen Orthesengelenkmechanik vermieden.

Erfindungsgemäß ist es zweckmäßig, wenn die die Dorsalierung des Unterschenkels über die Rückverlagerung der Gelenkschienen bewirkende Einrichtung eine ventrale elastische Zuggurtung ist, die um die seitlichen Gelenkschienen geführt ist. Die Zuggurtung kann erfindungsgemäß aus nur einem einzigen, um die beiden Gelenkschienen herumgeführten, längenveränderlichen Zuggurt bestehen, der aufgrund seiner ventralen Anordnung den Kniekehlenbereich völlig frei läßt und daher eine starke Beugung ermöglicht.

Grundsätzlich sind andere Ausführungen für die genannte Einrichtung denkbar. Beispielsweise kann die Zuggurtung über je ein längendehnbares Element, z. B. eine Zugfeder, an den Gelenkschienen angreifen. Die Einrichtung könnte auch als federbelasteter Schwenkhebel ausgebildet sein, dessen Drehpunkt im unteren Anlenkpunkt der Gelenkschiene liegt, und dessen freies Hebelende die Gelenkschiene an einem von dem Drehpunkt beabstandeten Punkt hintergreift.

Erfindungsgemäß wird eine solche Anordnung der beiden Anlenkpunkte einer Gelenkschiene vorgenommen, daß bei gestrecktem Bein die Gelenkschiene entsprechend der individuellen Beinachse angenähert lotrecht und angenähert mittig zur Sagittalebene des Beines liegt, während sich bei zunehmender Beugung des Knies die Gelenkschienen hinter den Kniedrehpunkt bewegen. Denn auch eine sagittale Beinachsfehlstellung wird entsprechend der individuellen Vorgaben aufgrund der drehbeweglichen Anlenkung der Gelenkschiene am proximalen Punkt in allen drei Raumebenen und durch die separate Längenanpassung der Gelenkschiene eingenommen. Das besondere an diesen Gelenkschienen ist darin zu sehen, daß sie vom anatomischen und funktionellen Drehmechanismus des Kniegelenkes unabhängig sind. Sie könnten grundsätzlich schon in der Ausgangsposition hinter dem Kniedrehpunkt liegen, was lediglich aus Praktikabilitätsgründen nicht realisiert wird.

Insbesondere das Anlegen der Orthese gestaltet sich dann besonders einfach und sicher, wenn das Oberschenkelteil und das Unterschenkelteil durch je eine den Schenkel weitgehend umgreifende Schale (obere Schale, untere Schale) gebildet ist.

Um eine sehr starke Beugung des Knies bis zur Anlage des Unterschenkels an den Oberschenkel zu ermöglichen, ist es zweckmäßig, wenn jede Schale dorsal im Bereich der Kniekehle einen Ausschnitt aufweist.

Um ein Verrutschen der oberen Schale aufgrund der konischen Anatomie des Oberschenkels nach unten zu verhindern, ist es vorteilhaft, wenn die obere Schale auf ihrer Innenseite oberhalb der innenseitigen Oberschenkelkondylen eine Kondylenbettung aufweist. Diese Kondylenbettung kann als separate, individuell positionierbare Pelotte ausgebildet sein, die sich z. B. über einen Klettverschluß an der gewünschten Position festlegen läßt. Es besteht aber auch die Möglichkeit, den entsprechenden Innenbereich der oberen Schale verformbar auszubilden, um ihn so an die individuellen Gegebenheiten anpassen zu können. Hierfür kann eine Wasser- oder Gasfüllung vorgesehen werden, die auch selbstregulierend ausgebildet werden kann.

Ebenfalls zur Lagesicherung der Schalen ist es vorteilhaft, wenn diese jeweils mit einer Innenauskleidung versehen sind.

Zur Erhöhung der Verwindungssteifigkeit des Unterschenkelteils gegenüber dem Oberschenkelteil ist es vorteilhaft, wenn die beiden unteren Anlenkpunkte der Gelenkschienen über einen dorsalen Bügel miteinander verbunden sind. Dieser Bügel kann in lichtem Abstand außen um den dorsalen Bereich der unteren Schale herumgeführt oder aber auch unmittelbar in die untere Schale integriert werden.

Weitere Merkmale der Erfindung sind Gegenstand der Unteransprüche und werden in Verbindung mit weiteren Vorteilen der Erfindung anhand eines Ausführungsbeispieles näher erläutert.

Grundsätzlich läßt sich der vorstehend für eine Kniegelenkorthese beschriebene Zügelungsmechanismus ohne feststehende Gelenkbewegungsachse auch für andere orthetische Gelenkversorgungen einsetzen, beispielsweise als Schultergelenkorthese.

In der Zeichnung ist eine als Beispiel dienende Ausführungsform der Erfindung schematisch dargestellt. Es zeigen:

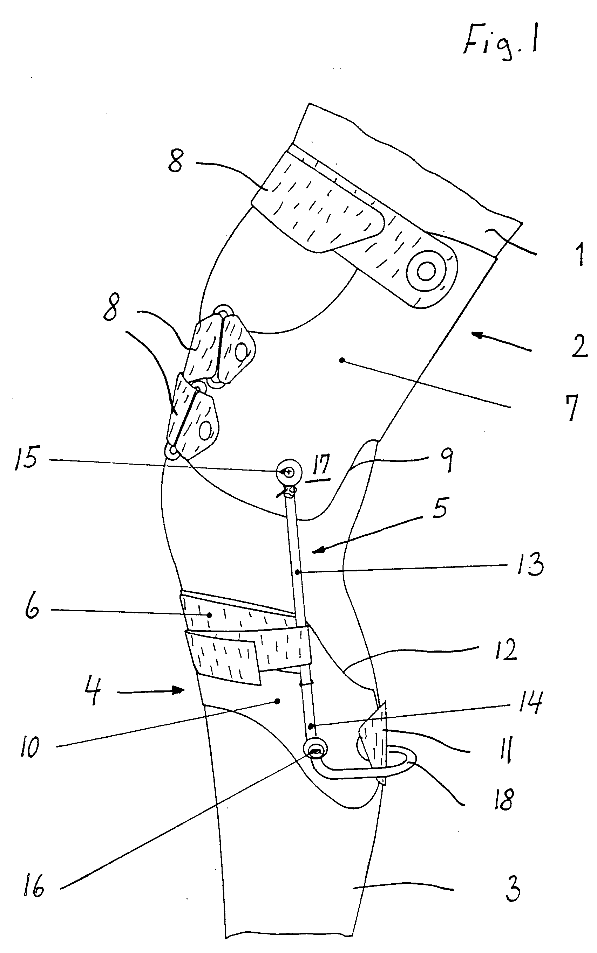

- **Figur 1 -**: in Seitenansicht eine an einem Bein festgelegte Kniegelenkorthese und

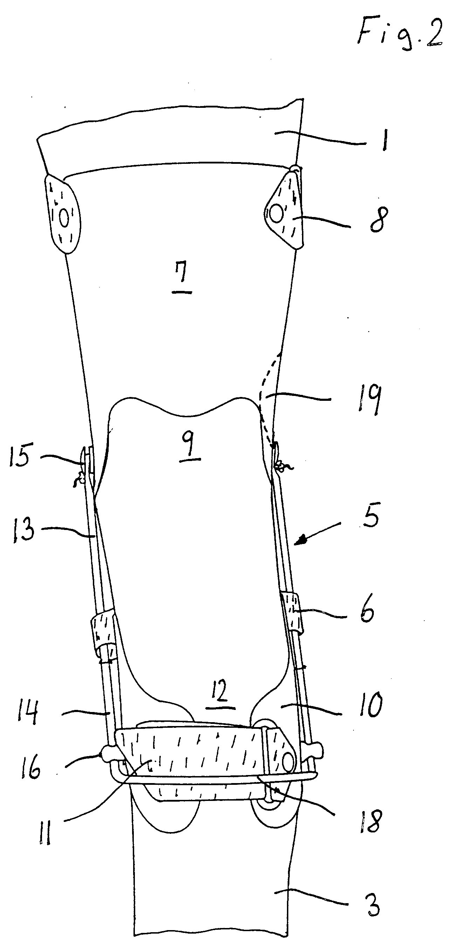

- **Figur 2 -**: die Darstellung gemäß **Figur 1** in Rückansicht.

Die dargestellte Kniegelenkorthese umfaßt ein am Oberschenkel 1 festzulegendes Oberschenkelteil 2, ein am Unterschenkel 3 festzulegendes Unterschenkelteil 4 und zwei seitlich gegenüberliegende, die beiden Schenkelteile 2,4 gelenkig miteinander verbindende Gelenkschienen 5. Vorgesehen ist ferner eine ventral um die Gelenkschienen 5 als Querverbinder herumgeführte elastische Zuggurtung 6.

Das Oberschenkelteil 2 wird durch eine den Oberschenkel 1 weitgehend umgreifende obere Schale 7 gebildet, die ventral offen und in diesem Bereich durch Klettverschlüsse 8 verschließbar ist und dorsal im Bereich der Kniekehle einen diesen Bereich freilassenden Ausschnitt 9 aufweist.

Das Unterschenkelteil 4 ist ebenfalls durch eine den Unterschenkel 3 weitgehend umgreifende untere Schale 10 gebildet, die dorsal offen und hier durch einen Klettverschluß 11 verschließbar ist und dorsal im Bereich der Kniekehle einen Ausschnitt 12 aufweist.

Erfindungsgemäß ist jede der beiden Gelenkschienen 5 teleskopförmig längenveränderlich ausgebildet. In dem dargestellten Ausführungsbeispiel besteht jede Gelenkschiene 5 aus einem oberen Teleskoprohr 13 und einer in diesem geführten unteren Teleskopstange 14, die über einen inneren, in der Zeichnung nicht näher dargestellten Gummizug miteinander verbunden sein können, der auch durch einen Streckanschlag ersetzt werden könnte. Das obere Ende des Teleskoprohres 13 ist an der oberen Schale 7 über einen Anlenkpunkt 15 angelenkt, während entsprechend das untere Ende der unteren Teleskopstange 14 über einen Anlenkpunkt 16 an der unteren Schale 10 angelenkt ist. Die durch die Anlenkschiene 5 gebildete Verbindung der beiden Anlenkpunkte 15,16 ist zwar längenveränderlich, im übrigen aber starr ausgebildet. Der obere Anlenkpunkt 15 der inneren Anlenkschiene 5 liegt im Kondylenbereich 17 der oberen Schale 7, während der untere Anlenkpunkt 14 im Wadenbereich der unteren Schale 10 liegt. Dabei sind die beiden Anlenkpunkte 15,16 jeder Gelenkschiene 5 so angeordnet, daß bei gestrecktem Bein die Gelenkschiene 5 angenähert lotrecht und angenähert mittig zur Sagittalebene des Beines liegt, während sich bei zunehmender Beugung des Knies die Gelenkschienen 5 hinter den Kniedrehpunkt bewegen.

Die beiden unteren Anlenkpunte 16 der Gelenkschienen 5 sind über einen dorsalen Bügel 18 miteinander verbunden.

Die Zuggurtung 6 besteht aus einem einzigen, um die beiden Gelenkschienen 5 herumgeführten Zuggurt, dessen beiden freien Enden beispielsweise über einen Klettverschluß längenveränderlich miteinander verbunden werden können.

Die obere Schale 7 weist auf ihrer Innenseite oberhalb der innenseitigen Oberschenkelkondylen eine Kondylenbettung 19 auf, die als separate, beispielsweise über einen Klettverschluß individuell positionierbare Pelotte ausgebildet sein kann, oder aber durch einen verformbaren Bereich gebildet ist, der mit einem Gas oder Fluid gefüllt sein kann und ggf. selbstregulierend ausgebildet ist. Dieser Bereich kann auch durch Silikonmasse gebildet werden. In jedem Fall dient die Kondylenbettung zur Abstütung und damit besseren Fixierung der oberen Schale 7, die aufgrund der konischen Anatomie des Oberschenkels 1 die Neigung hat, nach unten zu verrutschen. Die Kondylenbettung ist dabei so angeordnet, daß sie sich an den Oberschenkelkondylenbereich anatomisch anformt.

Zur Verhinderung der Lageverschiebung bzw. zur Lagesicherung der Schalen 7,10 weisen diese vorzugsweise eine in der Zeichnung nicht näher dargestellte Innenauskleidung auf, die aus rutschhemmendem Material bestehen kann.

Die Zuggurtung 6 ist so ausgebildet bzw. wird so eingestellt, daß sie bei gestrecktem Bein relativ entspannt ist, bei zunehmender Kniebeugung jedoch zu einer zunehmenden Dorsalisierung des Unterschenkels führt, also zu einer zunehmenden, am Unterschenkel angreifenden Zugkraft nach hinten.

Die dorsalen Ausschnitte 9,12 in den Schalen 7,10 bilden körpergerechte Beinausschnitte, durch die Druckstellen in der Kniekehle vermieden werden. Dadurch ist auch eine maximale Beugung möglich, da sich in Kniekehlennähe weder Zuggurtungen noch Schalenbereiche befinden.

Die erfindungsgemäße Kniegelenkorthese nimmt keinen Einfluß auf den individuellen, physiologischen Roll-Gleit-Mechanismus des Kniegelenkes, führt also auch nicht zur Desintegration des natürlichen Bewegungsablaufes. Bei Erhalt der ursprünglichen Mechanik wird lediglich die Sicherung des Schienbeinkopfes gegen eine Verschiebung nach vorne bei muskulärer Minderaktivität verhindert. Beim Stehen mit gestrecktem Bein sichert im wesentlichen die rückwärtige Oberschenkelmuskulatur ein Verschieben des Schienbeinkopes nach vorne, während das Kniescheibenband ohne Zug ist. Die erfindungsgemäß vorgesehene ventrale Zuggurtung übt bei Streckung des Kniegelenkes eine leichte Zugkraft aus und sorgt dadurch für ein gutes Anliegen der Orthese und entfaltet bei weiterer Beugung des Gelenkes eine zunehmende Zugkraft.

Die erfindungsgemäße Kniegelenkorthese besitzt somit kein mechanisches Gelenk, das die individuelle Kniegelenkmechanik nachzuahmen versucht. Der jeweilige Bewegungsmittelpunkt, mit dem auch seitlich unterschiedlichen Verlauf der Gelenkachse, kann sich entsprechend den individuellen anatomischen Verhältnissen zwischen den Anlenkpunkten der Gelenkschienen einstellen. Durch diese Verbindung zwischen Oberschenkel- und Unterschenkelteil 2,4 wird ein Abhebeln oder Verrutschen und Scheuern aufgrund einer eigenständigen Orthesengelenkmechanik vermieden. Die beiden voneinander an sich unabhängigen Teile 2,4 sind ortsständig am Bein befestigt. Eine geringe Lageverschiebung ergibt sich lediglich aufgrund der unvermeidbaren Volumenveränderungen am Ober- und Unterschenkel durch Muskelanspannung. Durch die besondere Anordnung der beiden Gelenkschienen 5 ist eine Haltekonstanz während der Durchbewegung gewährleistet.

## Patentansprüche

1. Kniegelenkorthese mit einem über Bandagen oder dergleichen am Oberschenkel (1) festzulegenden Oberschenkelteil (2), einem ebenfalls über Bandagen oder dergleichen am Unterschenkel (3) festzulegenden Unterschenkelteil (4), zwei sich seitlich gegenüberliegenden, die beiden Schenkelteile (2,4) gelenkig miteinander verbindenden Gelenkschienen (5), die teleskopförmig längenveränderlich ausgebildet und mit ihrem oberen Ende am Oberschenkelteil (2) angelenkt (15) sind und mit einer eine Dorsalierung des Unterschenkels (3) bewirkenden Einrichtung, **dadurch gekennzeichnet**, daß jede der beiden Gelenkschienen (5) mit ihrem unteren Ende am Unterschenkelteil (4) angelenkt (16) ist und daß die genannte Einrichtung derart gestaltet ist, daß die Dorsalierung über eine Rückverlagerung der Gelenkschienen (5) bewirkt wird.

2. Kniegelenkorthese nach Anspruch 1, **dadurch gekennzeichnet**, daß die genannte Einrichtung eine ventrale elastische Zuggurtung (6) ist, die um die seitlichen Gelenkschienen (5) geführt ist.

3. Kniegelenkorthese nach Anspruch 2, **dadurch gekennzeichnet**, daß die Zuggurtung (6) ein um beide Gelenkschienen (5) herumgeführter, längenveränderlich ausgebildeter Zuggurt ist.

4. Kniegelenkorthese nach Anspruch 1, 2 oder 3 **gekennzeichnet durch** eine solche Anordnung der beiden Anlenkpunkte (15,16) einer Gelenkschiene (5), daß bei gestrecktem Bein die Gelenkschiene (5) entsprechend der idividuellen Beinachse angenähert lotrecht und angenähert mittig zur Sagittalebene des Beines liegt, während sich bei zunehmender Beugung des Knies die Gelenkschienen (5) hinter den Kniedrehpunkt bewegen.

5. Kniegelenkorthese nach einem der vorhergehenden Ansprüche, **dadurch gekennzeichnet**, daß jede Gelenkschiene (5) aus zwei teleskopförmig ineinander geführten Teleskopstangen (13,14) besteht.

6. Kniegelenkorthese nach einem der vorhergehenden Ansprüche, **gekennzeichnet durch** eine elastische, die Gelenkschiene (5) permanent in Richtung ihrer Verkürzung beaufschlagende Einrichtung.

7. Kniegelenkorthese nach einem der vorhergehenden Ansprüche, **dadurch gekennzeichnet**, daß das Oberschenkelteil (2) und das Unterschenkelteil (4) durch je eine den Schenkel weitgehend umgreifende Schale (obere Schale 7, untere Schale 10) gebildet sind.

8. Kniegelenkorthese nach Anspruch 7, **dadurch gekennzeichnet**, daß jede Schale (7,10) dorsal im Bereich der Kniekehle einen Ausschnitt (9,12) aufweist.

9. Kniegelenkorthese nach Anspruch 7 oder 8, **dadurch gekennzeichnet**, daß die obere Schale (7) ventral durch Klettverschlüsse (8) verschließbar ist.

10. Kniegelenkorthese nach Anspruch 7, 8 oder 9, **dadurch gekennzeichnet**, daß die untere Schale (10) dorsal durch einen Klettverschluß (11) verschließbar ist.

11. Kniegelenkorthese nach einem der Ansprüche 7 bis 10, **dadurch gekennzeichnet**, daß der obere Anlenkpunkt (15) der inneren Gelenkschiene (5) im Kondylenbereich (17) der oberen Schale (7) liegt.

12. Kniegelenkorthese nach einem der Ansprüche 7 bis 11, **dadurch gekennzeichnet**, daß der untere Anlenkpunkt (14) der beiden Gelenkschienen (5) im Wadenbereich der unteren Schale (10) liegt.

13. Kniegelenkorthese nach einem der Ansprüche 7 bis 12, **dadurch gekennzeichnet**, daß die obere Schale (7) auf ihrer Innenseite oberhalb der innenseitigen Oberschenkelkondylen eine Kondylenbettung (19) aufweist.

14. Kniegelenkorthese nach Anspruch 13, **dadurch gekennzeichnet**, daß die Kondylenbettung (19) als separate, individuell positionierbare Pelotte ausgebildet ist.

15. Kniegelenkorthese nach Anspruch 13, **dadurch gekennzeichnet**, daß die Kondylenbettung (19) durch einen verformbaren Bereich gebildet ist.

16. Kniegelenkorthese nach einem der Ansprüche 7 bis 15, **dadurch gekennzeichnet**, daß jede Schale (7,10) zu ihrer Lagesicherung eine Innenauskleidung aufweist.

17. Kniegelenkorthese nach einem der vorhergehenden Ansprüche, **dadurch gekennzeichnet**, daß die beiden unteren Anlenkpunkte (16) der Gelenkschienen (5) über einen dorsalen Bügel (18) miteinander verbunden sind.

## Claims

1. Knee joint orthosis, having an upper leg part (2) to be fixed to the upper leg (1) by way of bandages or the like, a lower leg part (4) to be fixed to the lower leg (3), also by way of bandages or the like, two laterally opposing joint splints (5) which connect the two leg parts (2, 4) to one another in jointed manner, are constructed to be telescopically variable in length and are articulated (15) at their upper end to the upper leg part (2) and having a means bringing about dorsalization of the lower leg (3), characterized in that each of the two joint splints (5) is articulated (16) at its lower end to the lower leg part (4), and in that the said means is formed such that dorsalization is brought about by displacing to the rear the joint splints (5).

2. Knee joint orthosis according to Claim 1, characterized in that the said means is a ventral resilient tensioning belt arrangement (6) which is guided around the lateral joint splints (5).

3. Knee joint orthosis according to Claim 2, characterized in that the tensioning belt arrangement (6) is a tensioning belt which is guided around both joint splints (5) and is constructed to be variable in its length.

4. Knee joint orthosis according to Claim 1, 2 or 3, characterized by an arrangement of the two articulation points (15, 16) of a joint splint (5) such that, when the leg is straight, the joint splint (5) lies approximately vertical in accordance with the individual leg axis and approximately central with respect to the sagittal plane of the leg, whereas as the knee increasingly bends the joint splints (5) move behind the pivot point of the knee.

5. Knee joint orthosis according to one of the preceding claims, characterized in that each joint splint (5) comprises two telescopic rods (13, 14) guided telescopically one inside the other.

6. Knee joint orthosis according to one of the preceding claims, characterized by a resilient arrangement which permanently acts on the joint splint (5) in the direction of shortening it.

7. Knee joint orthosis according to one of the preceding claims, characterized in that the upper leg part (2) and the lower leg part (4) are each formed by a shell (upper shell 7, lower shell 10) which reaches substantially around the leg.

8. Knee joint orthosis according to Claim 7, characterized in that each shell (7, 10) has a cutout (9, 12) dorsally in the region of the knee hollow.

9. Knee joint orthosis according to Claim 7 or 8, characterized in that the upper shell (7) can be closed ventrally by hook-and-burr fastenings (8).

10. Knee joint orthosis according to Claim 7, 8 or 9, characterized in that the lower shell (10) can be closed dorsally by a hook-and-burr fastening (11).

11. Knee joint orthosis according to one of Claims 7 to 10, characterized in that the upper articulation point (15) of the inner joint splint (5) lies in the condylar region (17) of the upper shell (7).

12. Knee joint orthosis according to one of Claims 7 to 11, characterized in that the lower articulation point (14) of the two joint splints (5) lies in the calf region of the lower shell (10).

13. Knee joint orthosis according to one of Claims 7 to 12, characterized in that the upper shell (7) has a condyle bedding means on its inside above the inside upper leg condyle (19).

14. Knee joint orthosis according to Claim 13, characterized in that the condyle bedding means (19) is constructed as a separate wadding cushion which can be positioned individually.

15. Knee joint orthosis according to Claim 13, characterized in that the condyle bedding means (19) is formed by a deformable region.

16. Knee joint orthosis according to one of Claims 7 to 15, characterized in that each shell (7, 10) has an inner lining to safeguard its position.

17. Knee joint orthosis according to one of the preceding claims, characterized in that the two lower articulation points (16) of the joint splints (5) are connected to one another by way of a dorsal shackle (18).

## Revendications

1. Orthèse de l'articulation du genou, comportant une pièce de cuisse (2) à fixer à la cuisse (1) par bandage ou similaires, une pièce de jambe (4) à fixer à la jambe (3) également par bandage ou similaires, et deux rails articulés (5) situés l'un en face de l'autre dans le sens latéral et reliant l'une à l'autre de manière articulée les deux pièces (2, 4) de cuisse et de jambe, qui sont réalisés sous forme télescopiquement modifiable en longueur et sont articulés (15) par leurs extrémités supérieures sur la pièce de cuisse (2) et avec un dispositif assurant une dorsalisation de la jambe (3), caractérisée en ce que chacun des deux rails articulés (5) est articulé (16) par son extrémité inférieure sur la pièce de jambe (4), et en ce que le dispositif précité est agencé de telle manière que la dorsalisation soit assurée par un décalage dorsal des rails articulés (5).

2. Orthèse de l'articulation du genou selon la revendication 1, caractérisée en ce que ledit dispositif est une sangle élastique ventrale (6) qui est passée autour des rails articulés (5) latéraux.

3. Orthèse de l'articulation du genou selon la revendication 2, caractérisée en ce que la sangle (6) est une sangle de longueur modifiable passée autour des deux rails articulés (5).

4. Orthèse de l'articulation du genou selon la revendication 1, 2 ou 3, caractérisée par un agencement des deux points d'articulation (15, 16) d'un rail articulé (5) tel que, lorsque la jambe est étendue, le rail articulé (5) est approximativement vertical et approximativement au milieu par rapport au plan sagittal de la jambe, en fonction de l'axe individuel de la jambe, tandis que, lorsque la flexion du genou augmente, les rails articulés (5) se déplacent en arrière du centre de rotation du genou.

5. Orthèse de l'articulation du genou selon l'une des revendications précédentes, caractérisée en ce que chaque rail articulé (5) est constitué de deux tiges télescopiques (13, 14) guidées télescopiquement l'une dans l'autre.

6. Orthèse de l'articulation du genou selon l'une des revendications précédentes, caractérisée par un dispositif élastique sollicitant le rail articulé (5) de manière permanente dans le sens de son raccourcissement.

7. Orthèse de l'articulation du genou selon l'une des revendications précédentes, caractérisée en ce que la pièce de cuisse (2) et la pièce de jambe (4) sont formées chacune par une coquille (coquille supérieure 7, coquille inférieure 10) entourant largement la cuisse ou la jambe.

8. Orthèse de l'articulation du genou selon la revendication 7, caractérisée en ce que chaque coquille (7, 10) présente dorsalement dans la zone du jarret une découpe (9, 12).

9. Orthèse de l'articulation du genou selon la revendication 7 ou 8, caractérisée en ce que la coquille supérieure (7) peut être refermée ventralement par une fermeture à friction (8).

10. Orthèse de l'articulation du genou selon la revendication 7, 8 ou 9, caractérisée en ce que la coquille inférieure (10) peut être refermée dorsalement par une fermeture à friction (11).

11. Orthèse de l'articulation du genou selon l'une des revendications 7 à 10, caractérisée en ce que le point d'articulation supérieur (15) du rail articulé intérieur (5) est situé dans la zone condylienne (17) de la coquille supérieure (7).

12. Orthèse de l'articulation du genou selon l'une des revendications 7 à 11, caractérisée en ce que le point d'articulation inférieur (14) des deux rails articulés (5) est situé dans la zone du mollet de la coquille inférieure (10).

13. Orthèse de l'articulation du genou selon l'une des revendications 7 à 12, caractérisée en ce que la coquille supérieure (7) présente un revêtement condylien (19) sur sa face inférieure, au-dessus du condyle intérieur de la cuisse.

14. Orthèse de l'articulation du genou selon la revendication 13, caractérisée en ce que le revêtement condylien (19) présente la forme d'une pelote séparée pouvant être positionnée individuellement.

15. Orthèse de l'articulation du genou selon la revendication 13, caractérisée en ce que le revêtement condylien (19) est formé par une zone déformable.

16. Orthèse de l'articulation du genou selon l'une des revendications 7 à 15, caractérisée en ce que chaque coquille (7, 10) présente un revêtement intérieur pour son immobilisation en position.

17. Orthèse de l'articulation du genou selon l'une des revendications précédentes, caractérisée en ce que les deux points d'articulation inférieurs (16) des rails articulés (5) sont reliés l'un à l'autre par un arceau dorsal (18).
